(19) Europäisches Patentamt
European Patent Office
Office européen des brevets

(11) **EP 3 535 581 B1**

(12) **EUROPÄISCHE PATENTSCHRIFT**

(45) Veröffentlichungstag und Bekanntmachung des Hinweises auf die Patenterteilung:
**30.11.2022 Patentblatt 2022/48**

(21) Anmeldenummer: **17787367.6**

(22) Anmeldetag: **10.10.2017**

(51) Internationale Patentklassifikation (IPC):
**G01N 33/22** *(2006.01)* **C10L 3/06** *(2006.01)*
**G01N 9/32** *(2006.01)* **G01N 9/36** *(2006.01)*

(52) Gemeinsame Patentklassifikation (CPC):
**G01N 33/225; G01N 9/36;** C10L 3/08;
C10L 2290/60

(86) Internationale Anmeldenummer:
**PCT/EP2017/075798**

(87) Internationale Veröffentlichungsnummer:
**WO 2018/082875 (11.05.2018 Gazette 2018/19)**

(54) **VERFAHREN ZUM BESTIMMEN VON EIGENSCHAFTEN EINES KOHLENWASSERSTOFFHALTIGEN GASGEMISCHES UND VORRICHTUNG DAFÜR**

METHOD FOR DETERMINING PROPERTIES OF A HYDROCARBON-CONTAINING GAS MIXTURE AND DEVICE FOR THE SAME

PROCÉDÉ PERMETTANT DE DÉTERMINER DES PROPRIÉTÉS D'UN MÉLANGE GAZEUX CONTENANT DES HYDROCARBURES ET DISPOSITIF À CET EFFET

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **07.11.2016 DE 102016121226**

(43) Veröffentlichungstag der Anmeldung:
**11.09.2019 Patentblatt 2019/37**

(73) Patentinhaber:
• **Endress+Hauser Flowtec AG**
**4153 Reinach (CH)**
• **TrueDyne Sensors AG**
**4153 Reinach (CH)**

(72) Erfinder:
• **HUBER, Christof**
**3007 Bern (CH)**
• **REITH, Patrick**
**4053 Basel (CH)**
• **BADARLIS, Anastasios**
**4127 Birsfelden (CH)**

(74) Vertreter: **Hahn, Christian et al**
**Endress+Hauser Group Services (Deutschland)AG +Co. KG**
**Colmarer Straße 6**
**79576 Weil am Rhein (DE)**

(56) Entgegenhaltungen:
**EP-A1- 1 193 488       EP-A1- 2 993 472**
**EP-A2- 0 967 483       EP-A2- 2 042 850**
**WO-A1-2013/092104    DE-A1- 19 900 129**
**DE-B- 1 017 735        DE-B1- 2 928 739**
**GB-A- 2 296 091**

• **DVGW DEUTSCHER VEREIN DES GAS- UND WASSERFACHES E. V.: "Technische Regel - Arbeitsblatt DVGW G 260 (A) Gasbeschaffenheit", TECHNISCHE REGELN, März 2013 (2013-03), XP002775713, ISSN: 0176-3490**
• **DVGW DEUTSCHER VEREIN DES GAS-UND WASSERFACHES E V: "Technische Regel - Arbeitsblatt DVGW G 260 (A) Gasbeschaffenheit", DVGW-REGELWERK, DEUTSCHE VEREINIGUNG DES GAS- UND WASSERFACHES, DE, 1 March 2013 (2013-03-01), pages 1-29, XP009501519, ISSN: 0176-3490**

**Beschreibung**

Die vorliegende Erfindung betrifft ein Verfahren zum Bestimmen von Eigenschaften eines kohlenwasserstoffhaltigen Gasgemisches und eine Vorrichtung dafür. Die relevanten Gasgemische sind insbesondere Erdgas oder Biogas.

Erdgas ist ein fossiler Energieträger. Es hat eine lagerstättenabhängige Zusammensetzung. Der Hauptbestandteil von Erdgas ist Methan, mit einem molaren Anteil von beispielsweise 75 % bis 99 %. Häufig enthält Erdgas auch größere Anteile an Ethan (1 % bis 15 %), Propan (1 % bis 10 %), Butan und Ethen. Weitere Nebenbestandteile sind Schwefelwasserstoff, Stickstoff, Kohlenstoffdioxid und Wasserdampf.

Biogas ist ein brennbares Gasgemisch unterschiedlicher Zusammensetzung, das durch Vergärung von Biomasse jeder Art entsteht. Es enthält im Rohzustand insbesondere Methan (bis zu 60%) und Kohlenstoffdioxid als Hauptkomponenten. Weiterhin sind Stickstoff, Sauerstoff, Schwefelwasserstoff, Wasserdampf und Ammoniak enthalten. Schwefelwasserstoff und Ammoniak müssen vor dem Verbrennen bzw. vor dem Einspeisen ins Erdgasnetz entfernt werden. Ebenso ist es vorteilhaft Kohlenstoffdioxid vor der Verbrennung abzuscheiden.

Um Verbrennungsprozesse zuverlässig steuern zu können, ist es erforderlich, den aktuellen Brennwert eines Gasgemischs, das gerade einem Prozess zugeführt wird, schnell zu erkennen.

Als technisch relevantere Charakterisierung des Brennwerts dient der Wobbe-Index W, welcher definiert ist als der Quotient aus dem Brennwert pro Volumeneinheit H und der Wurzel aus der relativen Dichte. Die relative Dichte ist der Quotient aus der Dichte $\rho$ des Brenngases und der Dichte trockener Luft $\rho_0$ unter gleichen Druck- und Temperaturbedingungen:

$$W = \frac{H}{\sqrt{\frac{\rho}{\rho_0}}}$$

Gasgemische von Erdgas mit gleichem Wobbe-Index können beim Betrieb eines Brenners ohne weiteres ausgetauscht werden. Anstelle eines Vergleichs des Dichtequotienten kann auch der dazu äquivalente Koeffizient der mittleren molaren Massen des Gases und trockener Luft verwendet werden.

Die Patentschriften DE 69 231 977 T2 und US 5 311 447 A1 offenbaren Verfahren zur Bestimmung des Brennwerts eines Gasgemischs aus der Wärmeleitfähigkeit, der Wärmekapazität, der optischen Absorption und der Viskosität des Gasgemischs.

Die Veröffentlichung GB 2 296 091 A beschreibt ein Verfahren zur Bestimmung des Brennwerts bzw. des Wobbe-Indexes eines Gasgemischs auf Basis von dessen Wärmeleitfähigkeit, Dichte, Viskosität, und Schallgeschwindigkeit. Die MEMS AG bietet unter der Bezeichnung Gas QS einen Sensor an, der auf Basis der Wärmeleitfähigkeit, der Wärmekapazität und der Dichte eines Gasgemischs dessen Brennwert bzw. Wobbe-Index bestimmt.

Die Veröffentlichung WO 2017 063 795 A1 offenbart ein Verfahren zum Verfahren zum Bestimmen des Wobbe-Indexes bzw. des Brennwerts wowie des Inertgasanteils von Gasgemischen, welche insbesondere Erdgas oder Biogas aufweisen, auf der Basis der Viskosität und der Dichte bzw, Schallgeschwindigkeit.

Das Gasnetz dient jedoch mit steigender Tendenz als Energiespeicher für alternativ erzeugtes Gas aus "Power to Gas" ($H_2$) und "Biogas" ($CH_4 + CO_2$) welches mit Flüssiggas ($C_2H_6+C_3H_8$) angereichert wird. Damit verändert sich die Gaszusammensetzung im Netz signifikant. Die Gasqualität beim Verbraucher schwankt stark und es können schnelle Änderungen auftreten. Der Wasserstoffanteil kann bis zu 20% betragen. Der Wobbe-Index ist als Maß für eine gute Brennerregelung nur noch bedingt geeignet, da sich $H_2$ anders verhält als Erdgas.

Es besteht daher Bedarf an einem einfachen, robusten und zuverlässigen Verfahren und einer entsprechenden Messvorrichtungen zur Bestimmung von Eigenschaften eines Gasgemisches, insbesondere dessen Brennwert, wenn es Wasserstoff enthält. Es ist daher die Aufgabe der vorliegenden Erfindung, ein solches Verfahren und eine solche Vorrichtung bereitzustellen.

Die Aufgabe wird erfindungsgemäß gelöst durch das Verfahren gemäß dem unabhängigen Patentanspruch 1 und die Vorrichtung gemäß dem unabhängigen Patentanspruch 11.

Das erfindungsgemäße Verfahren zum Bestimmen von Eigenschaften eines kohlenwasserstoffhaltigen Gasgemisches, welches insbesondere Erdgas oder Biogas aufweist, umfasst:

Strömen Lassen des Gasgemischs durch eine Messanordnung;

Bestimmen eines druck- und temperaturabhängigen Wärmeleitfähigkeitswerts, eines zugehörigen Temperaturmesswerts und eines zugehörigen Druckmesswerts des strömenden Gasgemischs;

Bestimmen eines Wasserstoffanteils $X_{H2}$ des Gasgemischs auf Basis des Wärmeleitfähigkeitswerts, des zugehörigen Temperaturmesswerts und des zugehörigen Druckmesswerts;

Bestimmen eines druck- und temperaturabhängigen Dichtemesswerts eines zugehörigen Temperaturmesswerts und eines zugehörigen Druckmesswerts des strömenden Gasgemischs;

Ermitteln eines Werts für die mittlere molare Masse oder die Standarddichte des Gasgemischs auf Basis des Dichtemesswerts, des zugehörigen Temperaturmesswerts und des zugehörigen Druckmesswerts;

Ermitteln eines Werts für die mittlere molare Masse oder die Standarddichte eines um den Wasserstoffanteil reduzierten Restgasgemischs auf Basis der mittleren molaren Masse des Gasgemischs und des Wasserstoffanteils

Bestimmen eines druck- und temperaturabhängigen Viskositätsmesswerts, eines zugehörigen Temperaturmesswerts und eines zugehörigen Druckmesswerts des strömenden Gasgemischs;

Ermitteln eines Werts für den Wobbe-Index des Restgasgemischs auf Basis des Viskositätsmesswerts, des zugehörigen Druckmesswerts und des zugehörigen Temperaturmesswerts; und

Ermitteln eines ersten Werts $CV_W$ für den Brennwert des Restgasgemischs auf Basis der mittleren molaren Masse bzw. der Standarddichte des Restgasgemischs und des Wobbe-Indexes des Restgasgemischs.

In einer Weiterbildung der Erfindung umfasst das Verfahren weiterhin das Ermitteln des Inertgasanteils des Restgasgemischs auf Basis des ersten Werts für den Brennwert und der mittleren molaren Masse bzw. der Standarddichte des Restgasgemischs.

In einer Weiterbildung der Erfindung umfasst das Ermitteln des Inertgasanteils die folgenden Schritte:

Ermitteln eines zweiten Werts $CV_M$ für den Brennwert auf Basis der mittleren molaren Masse bzw. der Standarddichte des Restgasgemischs mittels einer Funktion, die unabhängig von der Viskosität bzw. vom Wobbe-Index ist; und Ermitteln des Inertgasanteils als Funktion des ersten Werts für den Brennwert des Restgasgemischs und des zweiten Werts für den Brennwert des Restgasgemischs.

Dem Bestimmen des Wärmeleitfähigkeitswerts $\lambda$ bei einem gegebenen Druck und einer gegebenen Temperatur folgt in einer Ausgestaltung der Erfindung eine Umrechnung in eine Standardwärmeleitfähigkeit $\lambda_{ref}$ bei Standardbedingungen, beispielsweise mit einem Polynom in $\lambda$, p und T, insbesondere

$$\lambda_{ref} = \Sigma \, A_i \cdot T^{ti} \cdot \lambda^{li} \cdot p^{vi},$$

wobei i= 0 ... k ein Index der Summanden ist, und die $A_i$ deren Koeffizienten sowie $t_i$, $l_i$ und $v_i$ ganzzahlige Exponenten sind.Beispielsweise gilt:

$$\lambda_{ref} = A_0 + A_1 \cdot T + A_2 \cdot T^2 + A_3 \cdot p + A_4 \cdot \lambda + A_5 \cdot \lambda \cdot T.$$

In einer Ausgestaltung der Erfindung wird der Wasserstoffanteil $X_{H2}$ als Funktion der Standardwärmeleitfähigkeit berechnet.

Das Bestimmen der mittleren molaren Masse M des Gasgemisches umfasst in einer Ausgestaltung der Erfindung das Berechnen der mittleren molaren Masse als Funktion der Dichte, des Drucks und der Temperatur

$$M = f\,(\rho, \, T, \, p)$$

beispielsweise mittels eines Polynoms in $\rho$, p und T, also

$$M = \Sigma \, B_i \cdot T^{ti} \cdot \rho^{ri} \cdot p^{vi},$$

wobei i= 0 ... k ein Index der Summanden ist, und die $B_i$ deren Koeffizienten sowie $t_i$, $r_i$ und $v_i$ ganzzahlige Exponenten sind.

Beispielsweise gilt:

$$B_0 + B_1 \cdot \rho \cdot T / p + B_2 \cdot \rho^2 \cdot T / p + B_3 \cdot \rho^2 / p + B_4 \cdot (\rho \cdot T / p)^2 + B_5 \cdot p,$$

Die mittlere molare Masse $M_R$ des Restgasgemischs lässt sich aus der mittleren molaren Masse und dessen Wasserstoffanteil $X_{H2}$ berechnen als:

$$M_R = (M - X_{H2} \cdot M_{H2}) / (1 - X_{H2}),$$

wobei $M_{H2}$ die molare Masse von Wasserstoff ist.

In einer Weiterbildung der Erfindung geht dem Ermitteln des Werts für den Wobbe-Indexes das Ermitteln eines Standardviskositätswerts des strömenden Gasgemischs voraus, den das strömende Gasgemisch bei einer Standardtemperatur und einem Standarddruck aufweisen würde, auf Basis des Viskositätsmesswerts, des zugehörigen Temperaturmesswerts und des zugehörigen Druckmesswerts, wobei das Ermitteln des Wobbe-Indexes des strömenden Gasgemischs mittels des Standardviskositätswerts des Gasgemischs erfolgt. Die Standardviskosität resp. Standardviskosität kann beispielsweise bestimmt werden mit einem Polynom in $\eta$, p, $\rho$ und T, also:

$$\eta_{ref} = \Sigma\, C_i \cdot T^{ti} \cdot p^{vi} \cdot \eta^{ni} \cdot \rho^{ri},$$

wobei i = 0 ... k ein Index der Summanden ist, und die $C_i$ deren Koeffizienten sowie $t_i$, $v_i$, $n_i$ und $r_i$ ganzzahlige Exponenten sind. Beispielsweise gilt:

$$\eta_{ref} = C_0 + C_1 \cdot \eta + C_2 \cdot p + C_3 \cdot T + C_4 \cdot T^2.$$

In einer Ausgestaltung dieser Weiterbildung der Erfindung wird der Wobbe-Index des mit einem Polynom, insbesondere einer quadratischen oder linearen Funktion des Standardviskositätswerts berechnet werden gemäß:

$$W = \Sigma\, A_i \cdot \eta_{ref}^{\,ni},$$

wobei i = 0 ... k ein Index der Summanden ist, und die $A_i$ deren Koeffizienten sowie $n_i$ ganzzahlige Exponenten sind.

Beispielsweise gilt:

$$W = A_0 + A_i\, \eta_{ref}.$$

Der erste Wert $CV_W$ für den Brennwert des Restgases kann gemäß einer Ausgestaltung der Erfindung als Produkt des Wobbe-Indexes mit der Wurzel aus dem Quotienten der mittleren molaren Masse des Restgases geteilt durch die mittlere molare Masse trockener Luft berechnet werden, also:

$$CV_w = W \cdot (M_R / M_{Luft})^{1/2}$$

Der zweite Wert $CV_M$ für den Brennwert des Restgases kann gemäß einer Ausgestaltung der Erfindung als, insbesondere lineare Funktion der mittleren molaren Masse M des Restgases berechnet werden, beispielsweise:

$$CV_M = D_0 + M_R \cdot D_1$$

wobei die $D_i$ Konstanten sind.

In einer Weiterbildung der Erfindung erfolgt das Ermitteln des Inertgasanteils $X_{inert\text{-}R\text{-}C}$ im Restgas anhand eines Verhältnisses zwischen dem zweiten Wert für den Brennwert des Restgases und dem ersten Wert für den Brennwert des Restgases, beispielsweise

$$X_{inert\text{-}R\text{-}C} = E \cdot (CV_M / CV_W - 1),$$

wobei E eine Konstante ist.

In einer Weiterbildung der Erfindung umfasst das Verfahren weiterhin das Ermitteln eines Werts für den Inertgasanteil $X_{inert}$ des strömenden Gasgemischs auf Basis des Werts für den Inertgasanteil des Restgasgemischs $X_{inert-R}$ und des Wasserstoffanteils $X_{H2}$, beispielsweise gemäß

$$X_{inert} = X_{inert-R} \cdot (1 - X_{H2}).$$

In einer Weiterbildung der Erfindung umfasst das Verfahren weiterhin das Ermitteln eines Werts für den Brennwert des strömenden Gasgemischs auf Basis des Werts für den Wasserstoffanteil, des spezifischen Brennwerts von Wasserstoff und des Brennwerts des Restgasgemischs, beipielsweise gemäß

$$CV_{total} = CV_W \cdot (1 - X_{H2}) + CV_{H2} \cdot X_{H2}.$$

In einer Weiterbildung der Erfindung erfolgt das Bestimmen des Inertgasanteils unter der Annahme, dass das Inertgas im Wesentlichen Kohlenstoffdioxyd und Stickstoff umfasst.

In einer Weiterbildung der Erfindung wird zum Ermitteln der molaren Masse des strömenden Gasgemischs zunächst ein Wert für die Standarddichtewert für die Dichte des strömenden Gases bei Standardbedingungen auf Basis des druck- und temperaturabhängigen Dichtemesswerts des zugehörigen Temperaturmesswerts und des zugehörigen Druckmesswerts des strömenden Gasgemischs ermittelt.

In einer Weiterbildung der Erfindung erfolgt die Bestimmung der Viskosität und der Dichte des Gasgemischs, mittels eines vibronischen Sensors, wobei der vibronische Sensor insbesondere ein MEMS-Sensor ist, welcher mindestens ein durchströmtes, schwingendes Messrohr und/oder mindestens einen vom strömenden Gasgemischs umgebenen Oszillator, insbesondere in Form mindestens eines schwingenden Kragträgers oder einer schwingenden Stimmgabel aufweist.

Die erfindungsgemäße Vorrichtung zum Bestimmen von Eigenschaften eines kohlenwasserstoffhaltigen Gasgemisches, insbesondere mit einem erfindungsgemäßen Verfahren umfasst: eine von dem Gasgemisch durchströmbare Messanordnung, mit einem Temperatursensor, einem Drucksensor und einem vibronischen Sensor zum Bestimmen eines Viskositätsmesswerts und ggf. eines Dichtemesswerts des strömenden Gasgemischs; einen Wärmeleitfähigkeitssensor; und eine Auswertungseinheit zum Berechnen von Eigenschaften des strömenden Gasgemischs.

In einer Weiterbildung der Erfindung ist der vibronische Sensor ein MEMS-Sensor, welcher mindestens ein durchströmbares, schwingfähiges Messrohr und/oder mindestens einen vom strömenden Gasgemisch umgebenen Oszillator, insbesondere in Form mindestens eines schwingfähigen Kragträgers oder einer schwingfähigen Stimmgabel aufweist.

Die Erfindung wird nun anhand der in den Zeichnungen dargestellten Ausführungsbeispiele näher erläutert.

Es zeigt:

Fig. 1: ein Diagramm typischer Zusammensetzungen von Brenngasen;

Fig. 2: ein Diagramm der Stantdardviskosität und der Standardwärmeleitfähigkeit von reinen Gasen als Funktion des Brennwerts;

Fig. 3a: ein Diagramm des Wasserstoffanteils von Gasgemischen als Funktion der Standardärmeleitfähigkeit;

Fig. 3b: ein Diagramm des auf Basis der Standardwärmeleitfähigkeit ermittelten Wasserstoffanteils von Gasgemischen über dem tatsächlichen Wasserstoffanteil;

Fig. 4a: ein Diagramm des Wobbe-Indexes von um den Wasserstoffanteil bereinigten Restgasgemischen über der Standardviskosität der den Wasserstoffanteil enthaltenden Gasgemische;

Fig. 4b: ein Diagramm des Energiegehalts von um den Wasserstoffanteil bereinigten Restgasgemischen auf Basis von deren in Fig. 4a dargestellten Wobbe-Index;

Fig. 5: ein Korrelationsdiagramm des tatsächlichen Gehalts an Kohlenstoffdioxid und Stickstoff verschiedener Restgasgemische über der Abweichung zwischen den mittels Korrelationsrechnung mit Berücksichtigung des Viskositätswerts ermittelten Brennwerten und den mittels Korrelationsrechnung ohne Berücksichtigung des Viskositätswerts ermittelten Brennwerten der Restgasmischungen;

Fig. 6a: ein Flussdiagramm eines Ausführungsbeispiels des erfindungsgemäßen Verfahrens;

Fig. 6b: ein Flussdiagramm eines optionalen Aspekts des Ausführungsbeispiels aus Fig. 6a;

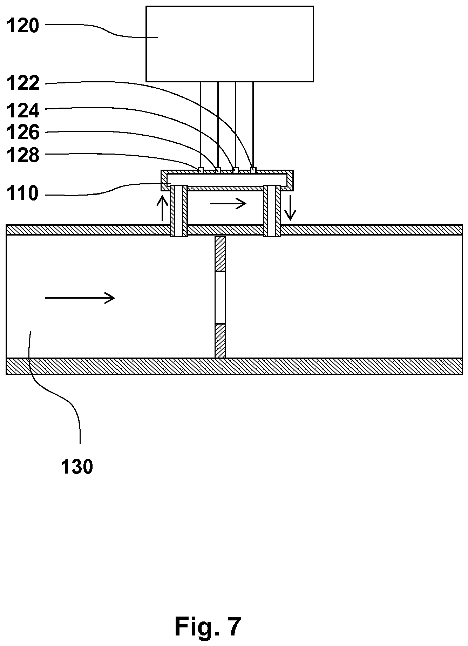

Fig. 7 eine Ausführungsbeispiel einer Vorrichtung zur Durchführung des erfindungsgemäßen Verfahrens.

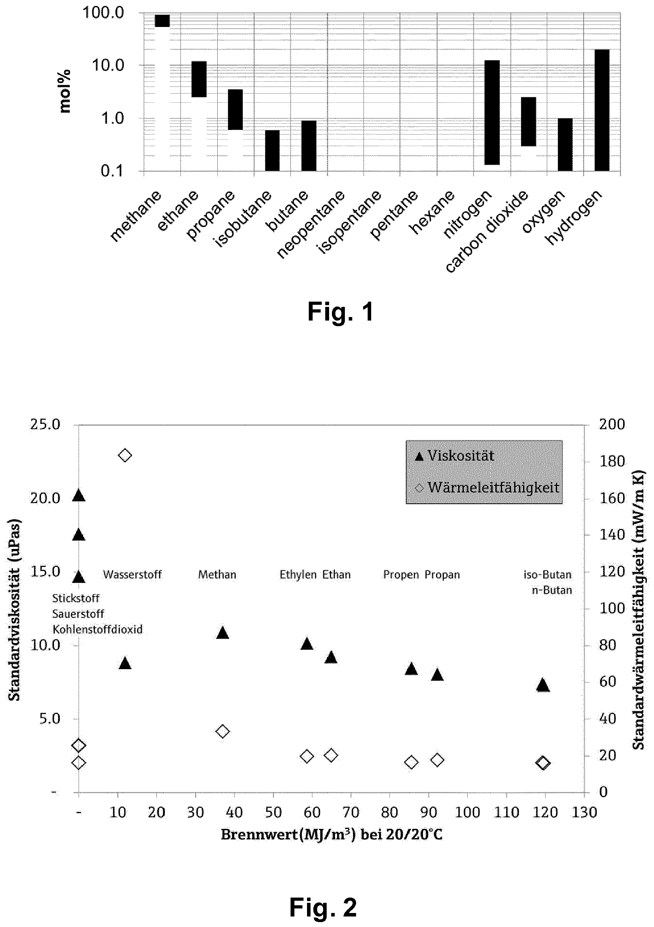

Die Erfindung beruht auf der statistischen Analyse der physikalischen Eigenschaften von mehreren hundert Proben von Gasgemischen, die Erdgas und Wasserstoff enthalten ergänzt durch im Bereich der in der Figur 1. dargestellten Zusammensetzungsbereiche zufällig generierten Gasgemische. Die jeweilige Zusammensetzung der Gasgemische wurde mittels Gaschromatographie bestimmt, oder zufällig generiert und für die ermittelten Zusammensetzungen wurden die physikalischen Eigenschaften der Gasgemische bei verschiedenen Druck- und Temperaturwerten rechnerisch ermittelt. Gleichermaßen wurden die physikalischen Eigenschaften einiger reiner Gase berechnet. Für die rechnerische Ermittlung der physikalischen Eigenschaften wurde ein Programm des NIST verwendet, nämlich "Reference Fluid Thermodynamic and Transport Properties Database", kurz REFPROP, Version 9.1, welches unter der Adresse http://www.nist.gov/srd/nist23.cfm zugänglich ist. Eine experimentelle Bestimmung der physikalischen Größen ist gleichermaßen möglich, bedeutet aber einen größeren Aufwand. Zu den rechnerisch ermittelten physikalischen Größen gehören:

- Dichte: $\rho(T.p)$

- Die mittlere Molare Masse: M

- Dynamische Viskosität: $\eta(T,p)$

- Oberer und unterer Brennwert: bzw. Brennwert und Heizwert CV

- Wobbe-Index: $WI = CV / (M/M_{Luft})^{0,5}$

Die auf Basis der obigen Daten durchgeführte Entwicklung des erfindungsgemäßen Verfahrens wird im Folgenden näher erläutert.

In Fig. 1 ist der Bereich von typischen molaren Anteilen von reinen Gasen in typischen Brenngasgemischen dargestellt. Es sind neben den kohlenwasserstoffhaltigen Gasen weiterhin Stickstoff, Kohlenstoffdioxyd, Sauerstoff und bis zu 20% Wasserstoff festzustellen.

In Fig. 2 sind die dynamische Viskosität und die Wärmeleitfähigkeit einiger reiner Gase über deren Brennwert dargestellt. Die Gase sind Stickstoff, Sauerstoff, Kohlenstoffdioxid, Wasserstoff, Methan, Ethylen, Ethan, Propen, Propan, iso-Butan, und n-Butan.

Es ist erkennbar, dass Gase allgemein mit geringerem Brennwert eine höhere dynamische Viskosität haben. Dies gilt jedoch nicht für Wasserstoff, der eine niedrigere Viskosität als Methan aufweist, obwohl sein Brennwert niedriger ist. Insofern kann die Viskosität nicht ohne weiteres als Messgröße zur Bestimmung des Brennwerts herangezogen werden. Andererseits weist Wasserstoff eine erheblich größere Wärmeleitfähigkeit auf als die anderen in Fig. 2 repräsentierten Gase. Damit bietet sich ein Ansatz anhand der Wärmeleitfähigkeit eines Gasgemischs dessen Wasserstoffanteil zu bestimmen, um den Wasserstoffanteil aus dem Gasgemisch herauszurechnen und anschließend für das Restgasgemisch auf Basis der Viskosität weitere Eigenschaften berechnen zu können, beispielsweise den Wobbe-Index oder den Brennwert.

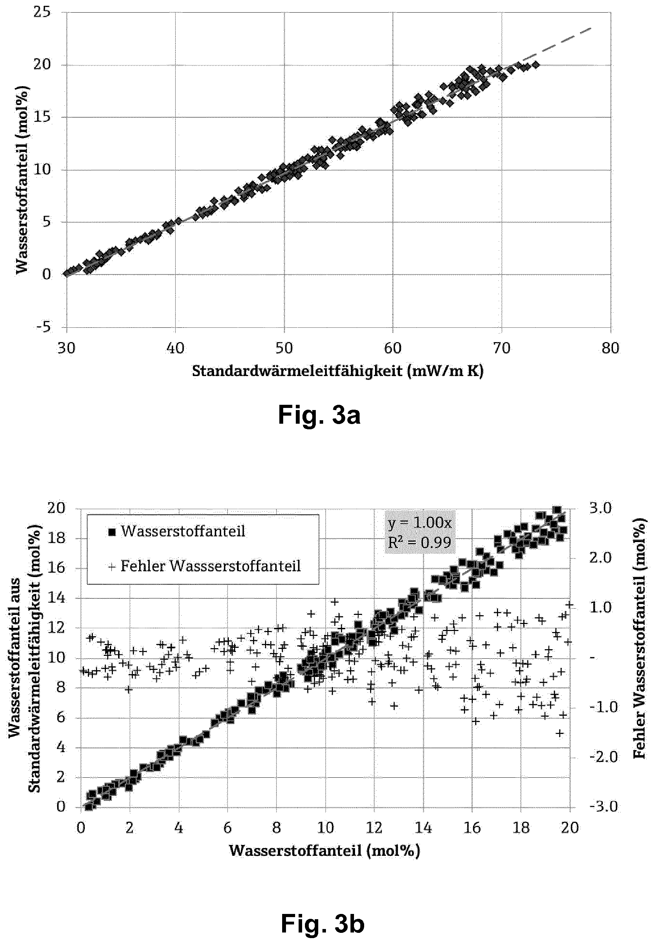

Wie in Fig. 3a dargestellt, korreliert der tatsächliche Anteil von Wasserstoff in Gemischen von Brenngasen mit der Wärmeleitfähigkeit. Der Wasserstoffanteil sollte daher mit hinreichender Genauigkeit als Funktion der Wärmeleitfähigkeit darstellbar sein, insbesondere als lineare Funktion der Wärmeleitfähigkeit, wobei letztere vorzugsweise zunächst als Wärmeleitfähigkeit bei Standardbedingungen berechnet wird gemäß einem Polynom in $\lambda$, p und T, insbesondere

$$\lambda_{ref} = A_0 + A_1 \cdot T + A_2 \cdot T^2 + A_3 \cdot p + A_4 \cdot \lambda + A_5 \cdot \lambda \cdot T, \qquad (1)$$

wobei $\lambda$ die bei p und T gemessene Wärmeleitfähigkeit ist, und die $A_i$ Konstanten sind.

Dies wird durch Fig. 3b bestätigt, in der die mittels der obigen Funktion auf Basis der Wärmeleitfähigkeit ermittelte Wasserstoffanteil sowie die Abweichung zwischen dem ermittelten Wasserstoffanteil und tatsächlichem Wasserstoffanteil jeweils über dem tatsächlichen Wasserstoffanteil dargestellt sind. Die Fehlerspanne ist für den Zweck der Charakterisierung eines Brenngases hinreichend klein.

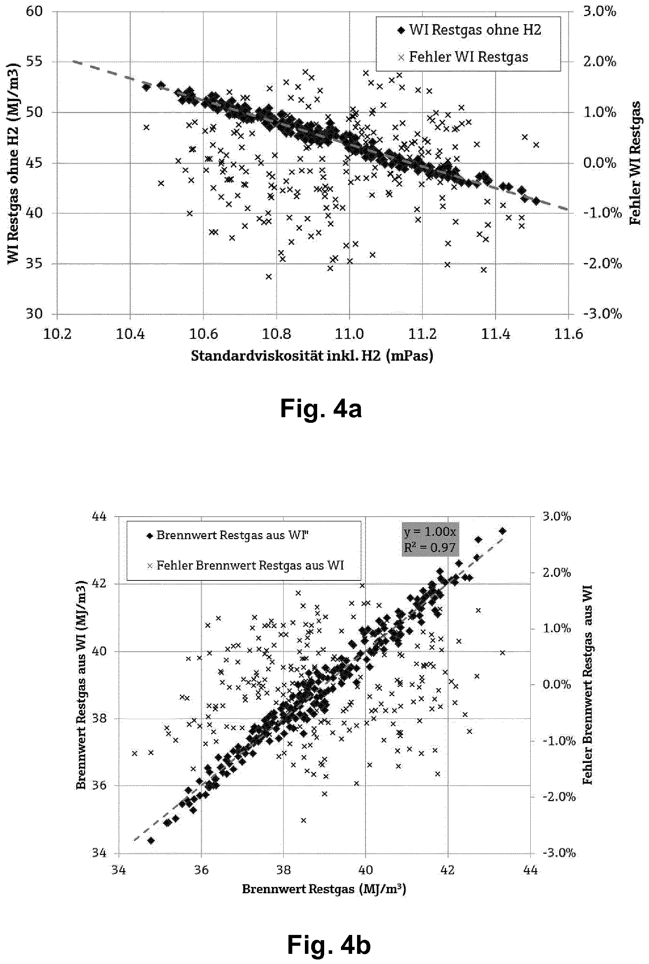

Wie schon in Patentanmeldung DE 10 2015 117 468.5 detailliert ausgeführt, korreliert der Wobbe-Index eines wasserstoffarmen Brenngasgemischs gut mit dessen Viskosität. Die Untersuchungen im Zusammenhang mit der vorliegenden Erfindung haben ergeben, dass die Viskositätswerte für wasserstoffhaltige Brenngasgemische mit dem Wobbe-Index der um den jeweiligen Wasserstoffanteil bereinigten Restgasgemische korrelieren, wie in Fig. 4 dargestellt ist. Damit lässt sich anhand der dynamischen Viskosität des gesamten strömenden Gasgemischs der Wobbe-Index des um den Wasserstoffanteils bereinigten Restgasgemischs ermitteln.

Es ist vorteilhaft, zunächst aus einem aktuellen Viskositätswert $\eta(T,p)$ bei einem gegebenen Druck $p$ und einer gegebenen Temperatur $T$ zunächst eine Standardviskosität bei Standardbedingungen $\eta_{ref}$ zu bestimmen, wobei dann der Wobbe Index auf Basis der Standardviskosität bei Standardbedingungen zu berechnen ist. Die Standardviskosität $\eta_{ref}$ ist aus einem aktuellen Viskositätswert beispielsweise zu berechnen mit einem Polynom in $\eta$, $p$ und $T$, insbesondere:

$$\eta_{ref} = C_0 + C_1 \cdot \eta + C_2 \cdot p + C_3 \cdot T + C_4 \cdot T^2 \qquad (2)$$

wobei die $C_i$ Konstanten sind.

Der Wobbe-Index $W$ für das Restgasgemisch wird dann als lineare Funktion der Standardviskosität bestimmt, also

$$W = A\,\eta_{ref} + B, \qquad (3)$$

wobei $A$ und $B$ Konstanten sind.

Aus dem auf Basis der Viskositätsmessung ermittelten Wobbe-Index für das um den Wasserstoffanteil bereinigten Restgasgemisch lässt sich durch Multiplikation mit der Wurzel aus dessen spezifischem Gewicht dessen Brennwert bestimmen, also

$$CV_w = W \cdot (M_R / M_{Luft})^{1/2} \qquad (4)$$

Die für die Berechnung erforderliche mittlere molare Masse $M_R$ des Restgasgemischs wird bestimmt anhand der Dichte des Gasgemischs und dessen Wasserstoffanteil.

$$M =$$

$$B_0 + B_1 \cdot \rho \cdot T / p + B_2 \cdot \rho^2 \cdot T / p$$
$$+ B_3 \cdot \rho^2 / p + B_4 \cdot (\rho \cdot T / p)^2 + B_5 \cdot p, \qquad (5)$$

wobei die $B_i$ Konstanten sind.

Die mittlere molare Masse $M_R$ des Restgasgemischs lässt sich aus der mittleren molaren Masse und dessen Wasserstoffanteil $X_{H2}$ berechnen als:

$$M_R = (M - X_{H2} \cdot M_{H2}) / (1 - X_{H2}), \qquad (6)$$

wobei $M_{H2}$ die molare Masse von Wasserstoff ist.

Mit der so ermittelten mittleren molaren Masse $M_R$ des Restgasgemischs kann nun dessen Brennwert auf $CV_w$ auf Basis des Wobbe-Indexes bestimmt werden gemäß Gleichung 2.

Andererseits zeigt der Brennwert eines Gasgemischs eine gute Korrelation zu dessen spezifischem Gewicht, solange die Inertgase Stickstoff und Kohlenstoffdioxid keine Rolle spielen. In diesem Fall kann der Brennwert $CV_M$ auf Basis der mittleren molaren Masse gut abgeschätzt werden mit

$$CV_M = D_0 + M \cdot D_1 \qquad (7)$$

wobei die $D_i$ Konstanten sind.

Kommen jedoch die Inertgase Stickstoff und Kohlenstoffdioxid hinzu, gilt die Korrelation nicht mehr und der Brennwert wird überschätzt. Dies macht sich die vorliegende Erfindung zu Nutze, um anhand der Abweichung zwischen dem Brennwert auf Basis der Viskosität und dem Brennwert ausschließlich auf Basis der mittleren molaren Masse eine den Anteil an Inertgasen $X_{inert-R-C}$ im Restgasgemischs zu berechnen gemäß

$$X_{inert-R-C} = E \cdot (CV_M / CV_W - 1), \tag{8}$$

wobei E eine Konstante ist

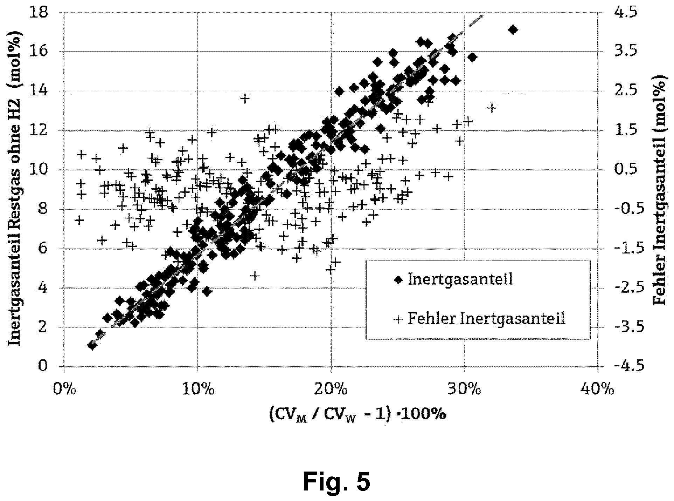

Anhand von Fig. 5 ist zu erkennen, dass dies ein valider Ansatz zur Bestimmung des Inertgasanteils $X_{inert-R}$ im Restgasgemisch ist. Die Rauten zeigen den tatsächlichen Inertgasanteil $X_{inert-R}$ über der Abweichung der Brennwerte des Restgasgemischs $CV_M / CV_W - 1$. Die Steigung der Im diagramm dargestellten Ausgleichsgeraden entspricht der obigen Konstante E. Die Kreuze zeigen den relativen Fehler, des gemäß der obigen Gleichung berechneten Inertgasanteils $X_{inert-R-C}$ jeweils bezogen auf den tatsächlichen $X_{inert-R}$. Im Ergebnis ist die vorgeschlagene Vorgehensweise zur Berechnung des Inertgasanteils zufriedenstellend. Der Inertgasanteil bezogen auf das gesamte Gasgemisch wird berechnet als

$$X_{inert} = X_{inert-R-C} \cdot (1 - X_{H2}). \tag{9}$$

Der Brennwert des gesamten Gasgemischs $CV_{total}$ wird berechnet gemäß

$$CV_{total} = CV_W \cdot (1 - X_{H2}) + CV_{H2} \cdot X_{H2}. \tag{10}$$

Mit den ermittelten Kenngrößen ist das Gasgemisch umfassend charakterisiert.

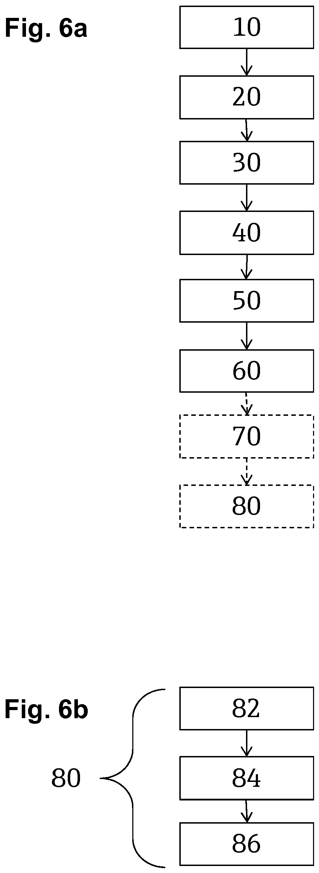

Zusammenfassend wird nun in Fig. 6 ein Flussdiagramme eines Ausführungsbeispiels des erfindungsgemäßen Verfahrens vorgestellt.

Ein in Fign. 6a und 6b dargestelltes Ausführungsbeispiel des erfindungsgemäßen Verfahrens umfasst in einem Schritt 10 das Erfassen eines Wärmeleitfähigkeitsmesswerts, eines Dichtemesswerts, eines Viskositätsmesswerts η, eines Temperaturmesswerts T und eines Druckmesswerts p des strömenden Gasgemischs, wobei die genannten Messwerte möglichst gleichzeitig zu erfassen sind und die dazu erforderlichen Sensoren vorzugsweise möglichst nahe beieinander angeordnet sind, so dass die Messwerte ein Wertetupel des Gasgemisches in einem thermodynamischen Zustand bilden. Die Messung der Viskosität und der Dichte erfolgt beispielsweise mit einem vibronischen Sensor, insbesondere einem schwingenden Cantilever, der von dem Gasgemisch umgeben ist.

In einem Schritt 20 wird auf Basis des Wärmeleitfähigkeitsmesswerts sowie der zugehörigen Druck- und Temperaturmesswerte der Wasserstoffanteil des Gasgemischs bestimmt, insbesondere als lineare Funktion der Wärmeleitfähigkeit bei Standardbedingungen, welche hier gemäß Gleichung 1 berechnet wird.

In einem Schritt 30 wird auf Basis des Dichtemesswerts sowie der zugehörigen Druck- und Temperaturmesswerte die mittlere molare Masse des Gasgemischs gemäß Gleichung 5 bestimmt.

In einem Schritt 40 wird auf Basis der mittleren molaren Masse des Gasgemischs und des Wasserstoffanteils die mittlere Molare Masse eines vom Wasserstoff befreiten Restgasgemischs berechnet.

In einem Schritt 50 wird auf Basis der Viskosität der Wobbe-Index des Restgasgemischs ermittelt.

In einem Schritt 60 wird auf Basis der mittleren molaren Masse des Restgasgemischs und des Wobbe-Indexes des Restgasgemischs der Brennwert des Restgasgemischs gemäß Gleichung 4 ermittelt.

Optional kann in einem Schritt 70 auf Basis des Brennwerts des Restgasgemischs und des Wasserstoffgehalts der Gesamtbrennwert des Gasgemischs gemäß Gleichung 10 ermittelt werden.

Als weitere Option kann in einem Schritt 80 der Inertgasanteil $X_{inert}$ des Gasgemischs ermittelt werden, Einzelheiten hierzu sind in dem Flussdiagram in Fig. 6b dargestellt.

Zunächst wird dazu in einem Schritt 82 ein zweiter Brennwert für das Restgasgemisch auf Basis der mittleren molaren Masse des Restgasgemischs gemäß Gleichung 7 ermittelt.

Anschließend wird in einem Schritt 84 auf Basis des Verhältnisses des zweiten Brennwerts für das Restgasgemisch zu dem über den Wobbe-Index ermittelten Brennwert der Inertgasanteil $X_{inert-R-C}$ des Restgasgemischs gemäß Gleichung 8 berechnet.

Daraus wird in einem Schritt 86 der Inertgasanteil des Gasgemischs gemäß Gleichung 9 berechnet.

Das Ausführungsbeispiel einer erfindungsgemäßen Vorrichtung zur Durchführung des erfindungsgemäßen Verfahrens umfasst eine von dem Gasgemisch durchströmbare Messzelle 110, in welcher hier nur schematisch dargestellte Sensorelemente, nämlich ein Cantileverschwinger 122 zum Bestimmen der Viskosität und der Dichte eines Gasgemischs in der Messzelle, ein Drucksensor 124, ein Temperatursensor 126, und ein Wärmeleitfähigkeitssensor 128 angeordnet sind. Die Sensorelemente sind vorzugsweise in MEMS-Technologie realisiert. Die einzelnen Sensorprinzipien sind dem Fachmann im vorliegenden Sachgebiet an sich bekannt und brauchen hier nicht näher erläutert zu werden. Die Vorrichtung umfasst weiterhin eine Betriebs- und Auswerteeinheit 120 zum Treiben der Sensorelemente, zum Auswerten von deren Signalen, um die primären Messgrößen, wie Viskosität, Druck, Temperatur, Wärmeleitfähigkeit und Dichte zu bestimmen, und zum Ermitteln der mittleren molaren Masse, des Wasserstoffanteils, des Wobbe Index und/oder des Brennwerts und/oder des Inertgasanteils eines die Messzelle 110 durchströmenden Gasgemischs. Die Betriebs- und Auswerteeinheit umfasst hierzu eine Recheneinheit, die kompakt oder modular aufgebaut sein kann, und insbesondere räumlich voneinander getrennte Module umfassen kann. Die Messzelle 110 ist insbesondere in einer Bypassanordnung an eine Gasleitung 130 angeschlossen, wobei ein Volumenstrom des Gasgemischs mittels einer Druckdifferenz über der Messzelle 110, beispielsweise aufgrund einer Blende bzw. einer Venturidüse in der Rohrleitung, oder mittels einer hier nicht dargestellten Pumpe durch die Messzelle 110 getrieben werden kann.

**Patentansprüche**

1. Verfahren zum Bestimmen von Eigenschaften eines kohlenwasserstoffhaltigen Gasgemisches, welches insbesondere Erdgas oder Biogas aufweist, umfassend:

   Strömen Lassen des Gasgemischs durch eine Messanordnung;
   Bestimmen eines druck- und temperaturabhängigen Wärmeleitfähigkeitswerts (10), eines zugehörigen Temperaturmesswerts und eines zugehörigen Druckmesswerts des strömenden Gasgemischs;
   Bestimmen eines Wasserstoffanteils (20) des Gasgemischs auf Basis des Wärmeleitfähigkeitswerts, des zugehörigen Temperaturmesswerts und des zugehörigen Druckmesswerts;
   Bestimmen eines druck- und temperaturabhängigen Dichtemesswerts (10) eines zugehörigen Temperaturmesswerts und eines zugehörigen Druckmesswerts des strömenden Gasgemischs;
   Ermitteln eines Werts für die mittlere molare Masse (30) oder der Standarddichte des Gasgemischs auf Basis des Dichtemesswerts, des zugehörigen Temperaturmesswerts und des zugehörigen Druckmesswerts;
   Ermitteln eines Werts für die mittlere molare Masse bzw. der Standarddichte eines um den Wasserstoffanteil reduzierten Restgasgemischs (40) auf Basis der mittleren molaren Masse oder der Standarddichte des Gasgemischs und des Wasserstoffanteils
   Bestimmen eines druck- und temperaturabhängigen Viskositätsmesswerts (10), eines zugehörigen Temperaturmesswerts und eines zugehörigen Druckmesswerts des strömenden Gasgemischs;
   Ermitteln eines Werts für den Wobbe-Index des Restgasgemischs (50) auf Basis des Viskositätsmesswerts, des zugehörigen Druckmesswerts und des zugehörigen Temperaturmesswerts;
   Ermitteln eines ersten Werts für den Brennwert (60) des Restgasgemischs auf Basis der mittleren molaren Masse bzw. der Standarddichte des Restgasgemischs und des Wobbe-Indexes des Restgasgemischs.

2. Verfahren nach Anspruch 1, weiterhin umfassend:
   das Ermitteln des Inertgasanteils des Restgasgemischs auf Basis des ersten Werts für den Brennwert und der mittleren molaren Masse bzw. der Standarddichte des Restgasgemischs.

3. Verfahren nach Anspruch 2, wobei das Ermitteln des Inertgasanteils die folgenden Schritte umfasst:

   Ermitteln eines zweiten Werts für den Brennwert (82) auf Basis der mittleren molaren Masse bzw. der Standarddichte des Restgasgemischs mittels einer Funktion, die unabhängig vom Wobbe-Index ist;
   Ermitteln des Inertgasanteils (84) als Funktion des ersten Werts für den Brennwert des Restgasgemischs und des zweiten Werts für den Brennwert des Restgasgemischs.

4. Verfahren nach Anspruch einem der vorhergehenden Ansprüche,wobei dem Ermitteln des Werts für den Wobbe-Index das Ermitteln eines Standardviskositätswerts des strömenden Gasgemischs voraus geht, den das strömende Gasgemisch bei einer Standardtemperatur und einem Standarddruck aufweisen würde, auf Basis des Viskositätsmesswerts, des zugehörigen Temperaturmesswerts und des zugehörigen Druckmesswerts, wobei das Ermitteln des Wobbe-Indexes mittels des Standardviskositätswerts des Gasgemischs erfolgt.

5. Verfahren nach Anspruch 3 oder 4, wobei das Ermitteln des Inertgasanteils anhand eines Verhältnisses zwischen dem zweiten Wert für den Brennwert des Restgases und dem ersten Wert für den Brennwert des Restgases erfolgt.

6. Verfahren nach einem der vorhergehenden Ansprüche, weiterhin umfassend:
   Ermitteln eines Werts für den Inertgasanteil des strömenden Gasgemischs (86) auf Basis des Werts für den Inertgasanteils des Restgasgemischs.

7. Verfahren nach einem der vorhergehenden Ansprüche, weiterhin umfassend:
Ermitteln eines Werts für den Brennwert des strömenden Gasgemischs (70) auf Basis des Werts für den Wasserstoffanteil, des spezifischen Brennwerts von Wasserstoff und des Brennwerts des Restgasgemischs.

8. Verfahren nach einem der vorhergehenden Ansprüche, wobei das Bestimmen des Inertgasanteils unter der Annahme erfolgt, dass das Inertgas im Wesentlichen Kohlenstoffdioxyd und Stickstoff umfasst.

9. Verfahren nach einem der vorhergehenden Ansprüche, wobei zum Ermitteln der molaren Masse des strömenden Gasgemischs zunächst ein Wert für die Standarddichte- des strömenden Gases bei Standardbedingungen auf Basis des druck-und temperaturabhängigen Dichtemesswerts des zugehörigen Temperaturmesswerts und des zugehörigen Druckmesswerts des strömenden Gasgemischs ermittelt wird.

10. Verfahren nach einem der vorhergehenden Ansprüche, wobei die Bestimmung der Viskosität und der Dichte des Gasgemischs, mittels eines vibronischen Sensors erfolgt, wobei der vibronische Sensor insbesondere ein MEMS-Sensor ist, welcher mindestens ein durchströmtes, schwingendes Messrohr und/oder mindestens einen vom strömenden Gasgemischs umgebenen Oszillator, insbesondere in Form mindestens eines schwingenden Kragträgers oder einer schwingenden Stimmgabel aufweist.

11. Vorrichtung zum Bestimmen von Eigenschaften eines kohlenwasserstoffhaltigen Gasgemisches mit einem Verfahren nach einem der vorhergehenden Ansprüche, wobei die Vorrichtung umfasst:

eine von dem Gasgemisch durchströmbare Messanordnung, mit einem Temperatursensor (126),
einem Drucksensor (124) und
einem vibronischen Sensor (122) zum Bestimmen eines Viskositätsmesswerts und eines Dichtemesswerts des strömenden Gasgemischs;
einen Wärmeleitfähigkeitssensor (128); und
eine Auswertungseinheit (120) zum Berechnen von Eigenschaften des strömenden Gasgemischs, wobei die Auswertungseinheit eingerichtet ist zum:

Bestimmen eines Wasserstoffanteils des Gasgemischs auf Basis des Wärmeleitfähigkeitswerts, des zugehörigen Temperaturmesswerts und des zugehörigen Druckmesswerts;
Ermitteln eines Werts für die mittlere molare Masse oder der Standarddichte des Gasgemischs auf Basis des Dichtemesswerts, des zugehörigen Temperaturmesswerts und des zugehörigen Druckmesswerts;
Ermitteln eines Werts für die mittlere molare Masse bzw. der Standarddichte eines um den Wasserstoffanteil reduzierten Restgasgemischs auf Basis der mittleren molaren Masse oder der Standarddichte des Gasgemischs und des Wasserstoffanteils;
Ermitteln eines Werts für den Wobbe-Index des Restgasgemischs auf Basis des Viskositätsmesswerts, des zugehörigen Druckmesswerts und des zugehörigen Temperaturmesswerts; und
Ermitteln eines ersten Werts für den Brennwert des Restgasgemischs auf Basis der mittleren molaren Masse bzw. der Standarddichte des Restgasgemischs und des Wobbe-Indexes des Restgasgemischs.

12. Vorrichtung nach Anspruch 11, wobei der vibronische Sensor (122) ein MEMS-Sensor ist. welcher mindestens ein durchströmbares, schwingfähiges Messrohr und/oder mindestens einen vom strömenden Gasgemisch umgebenen Oszillator, insbesondere in Form mindestens eines schwingfähigen Kragträgers oder einer schwingfähigen Stimmgabel aufweist.

## Claims

1. Procedure designed to determine properties of a gas mix containing hydrocarbons, wherein said gas mix particularly comprises natural gas or biogas, wherein said procedure comprises the following steps:

The gas mix is allowed to flow through a measuring arrangement;
Determination of a pressure- and temperature-dependent thermal conductivity value (10), an associated temperature measured value and an associated pressure measured value of the flowing gas mix;
Determination of a hydrogen proportion (20) of the gas mix on the basis of the thermal conductivity value, the associated temperature measured value and the associated pressure measured value;

Determination of a pressure- and temperature-dependent density measured value (10), an associated temperature measured value and an associated pressure measured value of the flowing gas mix;

Determination of a value for the average molar mass (30) or the standard density of the gas mix on the basis of the density measured value, the associated temperature measured value and the associated pressure measured value;

Determination of a value for the average molar mass or the standard density of a residual gas mix (40) reduced by the hydrogen proportion on the basis of the average molar mass or the standard density of the gas mix and the hydrogen proportion;

Determination of a pressure- and temperature-dependent viscosity measured value (10), an associated temperature measured value and an associated pressure measured value of the flowing gas mix;

Determination of a value for the Wobbe index of the residual gas mix (50) on the basis of the viscosity measured value, the associated pressure measured value and the associated temperature measured value;

Determination of a first value for the calorific value (60) of the residual gas mix on the basis of the average molar mass or the standard density of the residual gas mix and the Wobbe index of the residual gas mix.

2. Procedure as claimed in Claim 1, further comprising:
the determination of the inert gas proportion of the residual gas mix on the basis of the first value for the calorific value and the average molar mass or the standard density of the residual gas mix.

3. Procedure as claimed in Claim 2, wherein the determination of the inert gas proportion comprises the following steps:

Determination of a second value for the calorific value (82) on the basis of the average molar mass or the standard density of the residual gas mix using a function that is independent of the Wobbe index;

Determination of the inert gas proportion (84) as a function of the first value for the calorific value of the residual gas mix and the second value for the calorific value of the residual gas mix.

4. Procedure as claimed in one of the previous claims, wherein the determination of the value of the Wobbe index is preceded by the determination of a standard viscosity value for the flowing gas mix, which the flowing gas mix would present at a standard temperature and a standard pressure, on the basis of the viscosity measured value, the associated temperature measured value and the associated pressure measured value, wherein the Wobbe index is determined using the standard viscosity value of the gas mix.

5. Procedure as claimed in Claim 3 or 4, wherein the inert gas proportion is determined using a ratio between the second value for the calorific value of the residual gas and the first value for the calorific value of the residual gas.

6. Procedure as claimed in one of the previous claims, further comprising:
the determination of a value for the inert gas proportion of the flowing gas mix (86) on the basis of the value for the inert gas proportion of the residual gas mix.

7. Procedure as claimed in one of the previous claims, further comprising:
the determination of a value for the calorific value of the flowing gas mix (70) on the basis of the value for the hydrogen proportion, the specific calorific value of hydrogen and the calorific value of the residual gas mix.

8. Procedure as claimed in one of the previous claims, wherein the inert gas proportion is determined under the assumption that the inert gas essentially comprises carbon dioxide and nitrogen.

9. Procedure as claimed in one of the previous claims, wherein, to determine the molar mass of the flowing gas mix, a value is first determined for the standard density of the flowing gas under standard conditions on the basis of the pressure-and temperature-dependent density measured value of the associated temperature measured value and the associated pressure measured value of the flowing gas mix.

10. Procedure as claimed in one of the previous claims, wherein the viscosity and the density of the gas mix is determined using a vibronic sensor, the vibronic sensor particularly being a MEMS sensor, wherein said sensor has at least a vibrating measuring tube through which the medium flows and/or at least an oscillator surrounded by the flowing gas mix, particularly in the form of at least a vibrating cantilever beam or a vibrating tuning fork.

11. Device for determining properties of a gas mix containing hydrocarbon with a procedure as claimed in one of the previous claims, said device comprising:

a measuring arrangement through which the gas mix can flow, with a temperature sensor (126),
a pressure sensor (124) and
a vibronic sensor (122) for determining a viscosity measured value and a density measured value of the flowing gas mix;
a thermal conductivity sensor (128); and
an evaluation unit (120) for calculating properties of the flowing gas mix, wherein the evaluation unit is designed for the:

Determination of a hydrogen proportion of the gas mix on the basis of the thermal conductivity value, the associated temperature measured value and the associated pressure measured value;
Determination of a value for the average molar mass or the standard density of the gas mix on the basis of the density measured value, the associated temperature measured value and the associated pressure measured value;
Determination of a value for the average molar mass or the standard density of a residual gas mix reduced by the hydrogen proportion on the basis of the average molar mass or the standard density of the gas mix and the hydrogen proportion;
Determination of a value for the Wobbe index of the residual gas mix on the basis of the viscosity measured value, the associated pressure measured value and the associated temperature measured value; and
Determination of a first value for the calorific value of the residual gas mix on the basis of the average molar mass or the standard density of the residual gas mix and the Wobbe index of the residual gas mix.

12. Device as claimed in Claim 11, wherein the vibronic sensor (122) is a MEMS sensor that has at least a measuring tube which can vibrate and through which medium can flow and/or at least an oscillator surrounded by the flowing gas mix, particularly in the form of at least a cantilever beam which is capable of vibrating or a tuning fork which is capable of vibrating.

## Revendications

1. Procédé destiné à la détermination de propriétés d'un mélange gazeux contenant des hydrocarbures, lequel mélange gazeux comprend notamment du gaz naturel ou du biogaz, lequel procédé comprend les étapes suivantes :

Mise en écoulement du mélange gazeux à travers un dispositif de mesure ;
Détermination d'une valeur de conductivité thermique (10) dépendant de la pression et de la température, d'une valeur mesurée de température associée et d'une valeur mesurée de pression associée du mélange gazeux en écoulement ;
Détermination d'une proportion d'hydrogène (20) du mélange gazeux sur la base de la valeur de conductivité thermique, de la valeur mesurée de température associée et de la valeur mesurée de pression associée ;
Détermination d'une valeur mesurée de densité (10) dépendant de la pression et de la température, d'une valeur mesurée de température associée et d'une valeur mesurée de pression associée du mélange gazeux en écoulement ;
Détermination d'une valeur pour la masse molaire moyenne (30) ou la densité standard du mélange gazeux sur la base de la valeur mesurée de densité, de la valeur mesurée de température associée et de la valeur mesurée de pression associée ;
Détermination d'une valeur pour la masse molaire moyenne ou la densité standard d'un mélange gazeux résiduel (40) réduit de la proportion d'hydrogène sur la base de la masse molaire moyenne ou de la densité standard du mélange gazeux et de la proportion d'hydrogène ;
Détermination d'une valeur mesurée de viscosité (10) dépendant de la pression et de la température, d'une valeur mesurée de température associée et d'une valeur mesurée de pression associée du mélange gazeux en écoulement ;
Détermination d'une valeur pour l'indice de Wobbe du mélange gazeux résiduel (50) sur la base de la valeur mesurée de viscosité, de la valeur mesurée de pression associée et de la valeur mesurée de température associée ;
Détermination d'une première valeur pour le pouvoir calorifique (60) du mélange gazeux résiduel sur la base de la masse molaire moyenne ou de la densité standard du mélange gazeux résiduel et de l'indice de Wobbe du mélange gazeux résiduel.

2. Procédé selon la revendication 1, comprenant en outre :
la détermination de la proportion de gaz inerte du mélange gazeux résiduel sur la base de la première valeur pour le pouvoir calorifique et de la masse molaire moyenne ou de la densité standard du mélange gazeux résiduel.

3. Procédé selon la revendication 2, pour lequel la détermination de la proportion de gaz inerte comprend les étapes suivantes :

Détermination d'une deuxième valeur pour le pouvoir calorifique (82) sur la base de la masse molaire moyenne ou de la densité standard du mélange gazeux résiduel au moyen d'une fonction qui est indépendante de l'indice de Wobbe ;
Détermination de la proportion de gaz inerte (84) en fonction de la première valeur pour le pouvoir calorifique du mélange gazeux résiduel et de la deuxième valeur pour le pouvoir calorifique du mélange gazeux résiduel.

4. Procédé selon l'une des revendications précédentes, pour lequel la détermination de la valeur de l'indice de Wobbe est précédée par la détermination d'une valeur de viscosité standard du mélange gazeux en écoulement, valeur de viscosité que le mélange gazeux en écoulement présenterait à une température standard et à une pression standard, sur la base de la valeur mesurée de viscosité, de la valeur mesurée de température associée et de la valeur mesurée de pression associée, la détermination de l'indice de Wobbe étant effectuée au moyen de la valeur de viscosité standard du mélange gazeux.

5. Procédé selon la revendication 3 ou 4, pour lequel la détermination de la proportion de gaz inerte s'effectue à l'aide d'un rapport entre la deuxième valeur pour le pouvoir calorifique du gaz résiduel et la première valeur pour le pouvoir calorifique du gaz résiduel.

6. Procédé selon l'une des revendications précédentes, comprenant en outre :
la détermination d'une valeur pour la proportion de gaz inerte du mélange gazeux en écoulement (86) sur la base de la valeur pour la proportion de gaz inerte du mélange gazeux résiduel.

7. Procédé selon l'une des revendications précédentes, comprenant en outre :
la détermination d'une valeur pour le pouvoir calorifique du mélange gazeux en écoulement (70) sur la base de la valeur pour la proportion d'hydrogène, du pouvoir calorifique spécifique de l'hydrogène et du pouvoir calorifique du mélange gazeux résiduel.

8. Procédé selon l'une des revendications précédentes, pour lequel la détermination de la proportion de gaz inerte est effectuée en supposant que le gaz inerte comprend pour l'essentiel du dioxyde de carbone et de l'azote.

9. Procédé selon l'une des revendications précédentes, pour lequel, pour déterminer la masse molaire du mélange gazeux en écoulement, on détermine d'abord une valeur pour la densité standard du gaz en écoulement dans des conditions standard sur la base de la valeur mesurée de densité dépendant de la pression et de la température de la valeur mesurée de température associée et de la valeur mesurée de pression associée du mélange gazeux en écoulement.

10. Procédé selon l'une des revendications précédentes, pour lequel la détermination de la viscosité et de la densité du mélange gazeux est effectuée au moyen d'un capteur vibronique, le capteur vibronique étant notamment un capteur MEMS, lequel capteur présente au moins un tube de mesure vibrant traversé par le mélange gazeux et/ou au moins un oscillateur entouré par le mélange gazeux en écoulement, notamment sous la forme d'au moins un support en porte-à-faux vibrant ou d'un diapason vibrant.

11. Dispositif destiné à la détermination des propriétés d'un mélange gazeux hydrocarboné avec un procédé selon l'une des revendications précédentes, le dispositif comprenant :

un dispositif de mesure pouvant être traversé par le mélange gazeux, comprenant un capteur de température (126),
un capteur de pression (124) et
un capteur vibronique (122) destiné à la détermination d'une valeur mesurée de viscosité et d'une valeur mesurée de densité du mélange gazeux en écoulement ;
un capteur de conductivité thermique (128) ; et
une unité d'évaluation (120) destinée au calcul des propriétés du mélange gazeux en écoulement, l'unité d'évaluation étant conçue pour la :

Détermination d'une proportion d'hydrogène du mélange gazeux sur la base de la valeur de conductivité thermique, de la valeur mesurée de température associée et de la de mesurée de pression associée;

Détermination d'une valeur pour la masse molaire moyenne ou la densité standard du mélange gazeux sur la base de la valeur mesurée de densité, de la valeur mesurée de température associée et de la valeur mesurée de pression associée ;

Détermination d'une valeur pour la masse molaire moyenne ou la densité standard d'un mélange gazeux résiduel réduit de la proportion d'hydrogène sur la base de la masse molaire moyenne ou de la densité standard du mélange gazeux et de la proportion d'hydrogène ;

Détermination d'une valeur pour l'indice de Wobbe du mélange gazeux résiduel sur la base de la valeur mesurée de viscosité, de la valeur mesurée de pression associée et de la valeur mesurée de température associée ; et

Détermination d'une première valeur pour le pouvoir calorifique du mélange gazeux résiduel sur la base de la masse molaire moyenne ou de la densité standard du mélange gazeux résiduel et de l'indice de Wobbe du mélange gazeux résiduel.

12. Dispositif selon la revendication 11, pour lequel le capteur vibronique (122) est un capteur MEMS qui présente au moins un tube de mesure vibrant pouvant être traversé et/ou au moins un oscillateur entouré par le mélange gazeux en écoulement, notamment sous la forme d'au moins un support en porte-à-faux vibrant ou d'un diapason vibrant.

Fig. 1

Fig. 2

**Fig. 3a**

**Fig. 3b**

**Fig. 4a**

**Fig. 4b**

Fig. 5

**Fig. 6a**

```
┌─────────────┐
│     10      │
└─────────────┘
       │
       ▼
┌─────────────┐
│     20      │
└─────────────┘
       │
       ▼
┌─────────────┐
│     30      │
└─────────────┘
       │
       ▼
┌─────────────┐
│     40      │
└─────────────┘
       │
       ▼
┌─────────────┐
│     50      │
└─────────────┘
       │
       ▼
┌─────────────┐
│     60      │
└─────────────┘
       ┊
       ▼
┌ ─ ─ ─ ─ ─ ─ ┐
      70
└ ─ ─ ─ ─ ─ ─ ┘
       ┊
       ▼
┌ ─ ─ ─ ─ ─ ─ ┐
      80
└ ─ ─ ─ ─ ─ ─ ┘
```

**Fig. 6b**

80

```
┌─────────────┐
│     82      │
└─────────────┘
       │
       ▼
┌─────────────┐
│     84      │
└─────────────┘
       │
       ▼
┌─────────────┐
│     86      │
└─────────────┘
```

120

122
124
126
128

110

130

**Fig. 7**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 69231977 T2
- US 5311447 A1
- GB 2296091 A
- WO 2017063795 A1
- DE 102015117468